(19) **Europäisches Patentamt / European Patent Office / Office européen des brevets**

(11) **EP 2 014 299 A1**

(12) # EUROPEAN PATENT APPLICATION

(43) Date of publication:
**14.01.2009 Bulletin 2009/03**

(51) Int Cl.:
*A61K 38/36* (2006.01)    *A61P 7/04* (2006.01)

(21) Application number: **07112277.4**

(22) Date of filing: **11.07.2007**

| | |
|---|---|
| (84) Designated Contracting States:<br>**AT BE BG CH CY CZ DE DK EE ES FI FR GB GR HU IE IS IT LI LT LU LV MC MT NL PL PT RO SE SI SK TR**<br>Designated Extension States:<br>**AL BA HR MK RS** | (72) Inventor: **The designation of the inventor has not yet been filed**<br><br>Remarks:<br>The sequence listing, which is published as annex to the application documents, was filed after the date of filing. The applicant has declared that it does not include matter which goes beyond the content of the application as filed. |
| (71) Applicant: **NOVO NORDISK A/S**<br>**2880 Bagsvaerd (DK)** | |

(54) **Subcutaneous administration of coagulation factor VII**

(57)    The invention relates to the use of a Factor VIIa polypeptide for the manufacture of a medicament for treatment of a condition affectable by Factor VIIa, said medicament being for subcutaneous or intramuscular administration.

EP 2 014 299 A1

SEQUENCE LISTING

<110> Novo Nordisk A/S

<120> SUBCUTANEOUS ADMINISTRATION OF COAGULATION FACTOR VII

<130> 7698-000-EP

<160> 1

<170> PatentIn version 3.4

<210> 1
<211> 406
<212> PRT
<213> Homo sapiens

<220>
<221> MOD_RES
<222> (6)..(6)
<223> GAMMA-CARBOXYGLUTAMIC ACID

<220>
<221> MOD_RES
<222> (7)..(7)
<223> GAMMA-CARBOXYGLUTAMIC ACID

<220>
<221> MOD_RES
<222> (14)..(14)
<223> GAMMA-CARBOXYGLUTAMIC ACID

<220>
<221> MOD_RES
<222> (16)..(16)
<223> GAMMA-CARBOXYGLUTAMIC ACID

<220>
<221> MOD_RES
<222> (19)..(19)
<223> GAMMA-CARBOXYGLUTAMIC ACID

<220>
<221> MOD_RES
<222> (20)..(20)
<223> GAMMA-CARBOXYGLUTAMIC ACID

<220>
<221> MOD_RES
<222> (25)..(25)
<223> GAMMA-CARBOXYGLUTAMIC ACID

<220>
<221> MOD_RES
<222> (26)..(26)
<223> GAMMA-CARBOXYGLUTAMIC ACID

```
<220>
<221>  MOD_RES
<222>  (29)..(29)
<223>  GAMMA-CARBOXYGLUTAMIC ACID

<220>
<221>  MOD_RES
<222>  (35)..(35)
<223>  GAMMA-CARBOXYGLUTAMIC ACID

<400>  1
```

Ala Asn Ala Phe Leu Glu Glu Leu Arg Pro Gly Ser Leu Glu Arg Glu
1               5                   10                  15

Cys Lys Glu Glu Gln Cys Ser Phe Glu Glu Ala Arg Glu Ile Phe Lys
            20                  25                  30

Asp Ala Glu Arg Thr Lys Leu Phe Trp Ile Ser Tyr Ser Asp Gly Asp
        35                  40                  45

Gln Cys Ala Ser Ser Pro Cys Gln Asn Gly Gly Ser Cys Lys Asp Gln
    50                  55                  60

Leu Gln Ser Tyr Ile Cys Phe Cys Leu Pro Ala Phe Glu Gly Arg Asn
65                  70                  75                  80

Cys Glu Thr His Lys Asp Asp Gln Leu Ile Cys Val Asn Glu Asn Gly
                85                  90                  95

Gly Cys Glu Gln Tyr Cys Ser Asp His Thr Gly Thr Lys Arg Ser Cys
            100                 105                 110

Arg Cys His Glu Gly Tyr Ser Leu Leu Ala Asp Gly Val Ser Cys Thr
        115                 120                 125

Pro Thr Val Glu Tyr Pro Cys Gly Lys Ile Pro Ile Leu Glu Lys Arg
        130                 135                 140

Asn Ala Ser Lys Pro Gln Gly Arg Ile Val Gly Gly Lys Val Cys Pro
145                 150                 155                 160

Lys Gly Glu Cys Pro Trp Gln Val Leu Leu Leu Val Asn Gly Ala Gln
                165                 170                 175

```
Leu Cys Gly Gly Thr Leu Ile Asn Thr Ile Trp Val Val Ser Ala Ala
            180                 185                 190

His Cys Phe Asp Lys Ile Lys Asn Trp Arg Asn Leu Ile Ala Val Leu
            195                 200                 205

Gly Glu His Asp Leu Ser Glu His Asp Gly Asp Glu Gln Ser Arg Arg
            210                 215                 220

Val Ala Gln Val Ile Ile Pro Ser Thr Tyr Val Pro Gly Thr Thr Asn
225                 230                 235                 240

His Asp Ile Ala Leu Leu Arg Leu His Gln Pro Val Val Leu Thr Asp
                245                 250                 255

His Val Val Pro Leu Cys Leu Pro Glu Arg Thr Phe Ser Glu Arg Thr
            260                 265                 270

Leu Ala Phe Val Arg Phe Ser Leu Val Ser Gly Trp Gly Gln Leu Leu
            275                 280                 285

Asp Arg Gly Ala Thr Ala Leu Glu Leu Met Val Leu Asn Val Pro Arg
            290                 295                 300

Leu Met Thr Gln Asp Cys Leu Gln Gln Ser Arg Lys Val Gly Asp Ser
305                 310                 315                 320

Pro Asn Ile Thr Glu Tyr Met Phe Cys Ala Gly Tyr Ser Asp Gly Ser
                325                 330                 335

Lys Asp Ser Cys Lys Gly Asp Ser Gly Gly Pro His Ala Thr His Tyr
            340                 345                 350

Arg Gly Thr Trp Tyr Leu Thr Gly Ile Val Ser Trp Gly Gln Gly Cys
            355                 360                 365

Ala Thr Val Gly His Phe Gly Val Tyr Thr Arg Val Ser Gln Tyr Ile
            370                 375                 380

Glu Trp Leu Gln Lys Leu Met Arg Ser Glu Pro Arg Pro Gly Val Leu
385                 390                 395                 400

Leu Arg Ala Pro Phe Pro
```

405

**Description**

**FIELD OF THE INVENTION**

The invention relates to the use of Factor VIIa polypeptides for the manufacture of a medicament for treatment of conditions affectable by Factor VIIa, wherein the medicament is for subcutaneous or intramuscular administration.

**BACKGROUND OF INVENTION**

Blood coagulation is a process consisting of a complex interaction of various blood components, or factors, which eventually gives rise to a fibrin clot. Generally, the blood components that participate in what has been referred to as the coagulation "cascade" are proenzymes or zymogens, enzymatically inactive proteins that are converted to proteolytic enzymes by the action of an activator, which is itself an activated clotting Factor. Coagulation factors that have undergone such a conversion are generally referred to as "active factors", and are designated by the addition of a lower case "a" suffix (e.g., Factor VIIa).

Activated Factor X ("Xa") is required to convert prothrombin to thrombin, which then converts fibrinogen to fibrin as a final stage in forming a fibrin clot. There are two systems, or pathways, that promote the activation of Factor X. The "intrinsic pathway" refers to those reactions that lead to thrombin formation through utilisation of factors present only in plasma. A series of protease-mediated activations ultimately generates Factor IXa, which, in conjunction with Factor VIIIa, cleaves Factor X into Xa. An identical proteolysis is effected by Factor VIIa and its co-Factor, tissue factor, in the "extrinsic pathway" of blood coagulation. Tissue factor is a membrane bound protein and does not normally circulate in plasma. Upon vessel disruption, however, it can complex with Factor VIIa to catalyse Factor X activation or Factor IX activation in the presence of $Ca^{++}$ and phospholipid. While the relative importance of the two coagulation pathways in haemostasis is unclear, in recent years Factor VII and tissue factor have been found to play a pivotal role in the regulation of blood coagulation.

Factor VII is a trace plasma glycoprotein that circulates in blood as a single-chain zymogen. The zymogen is catalytically inactive. Single-chain Factor VII may be converted to two-chain Factor VIIa by Factor Xa, Factor XIIa, Factor IXa or thrombin *in vitro.* Factor Xa is believed to be the major physiological activator of Factor VII. Like several other plasma proteins involved in haemostasis, Factor VII is dependent on vitamin K for its activity, which is required for the γ-carboxylation of multiple glutamic acid residues that are clustered in the amino terminus of the protein. These γ-carboxylated glutamic acids are required for the metal-associated interaction of Factor VII with phospholipids.

The conversion of zymogen Factor VII into the activated two-chain molecule occurs by cleavage of an internal peptide bond located approximately in the middle of the molecule. In human Factor VII, the activation cleavage site is at $Arg_{152}$-$Ile_{153}$. In the presence of tissue factor, phospholipids and calcium ions, the two-chain Factor VIIa rapidly activates Factor X or Factor IX by limited proteolysis.

Recombinant human Factor VIIa (rFVIIa) is an activated coagulation factor that is useful in the treatment of haemophilia patients who generate neutralising antibodies against Factor VIII or Factor IX. Administration of Factor VIII and Factor IX causes severe antibody formation in approximately 30% of the severe haemophilia patients. The action of rFVIIa (activation of the coagulation system via Factor X) is exerted in the vascular compartment of the body. The route of administration of rFVIIa has until now been intravenously. An alternative form of administration which would result in a reasonable bioavailability would allow an increase in dosing intervals and at the same time make self administration possible, thus increasing the convenience for the patient.

Factor VIIa is a glycoprotein with a molecular weight of approximately 50 kDa. Factor VIIa has conventionally been delivered intravenously to haemophilia A or B patients, either prophylactically (such as, e.g., in anticipation of planned surgery) or in response to bleeding episodes. Such repeated use of intravenous injections, while necessary to control the disease, may have side effects. Repeated injections may lead to the vein at the site of injection becoming fibrosed or occluded, a problem especially acute when treating the elderly. Also, when veins are small, as in babies/infants/toddlers, it may be difficult for the doctor/parents to insert a needle into the vein to inject the required therapeutic dose. A further impediment to intravenous administration is the prolonged time required for the infusion, which can be problematic when the patient is a child.

Coagulation Factors VIII (170 - 300 kDa) and IX (60 kDa) may be administered subcutaneously in the form of the single-chain zymogens, i.e., polypeptides not yet been activated. These non-activated forms are more stable than the activated (cleaved) forms, which are degraded much faster.

Factor VIIa is useful for administration to mammals, particularly humans, to control bleeding disorders, particularly bleeding disorders which are caused by clotting factor deficiencies (haemophilia A and B), or clotting factor inhibitors or bleeding disorders in patients not suffering from haemophilia A or B, for example, in patients suffering from von Willebrand's disease. Patients with von Willebrand's disease have a defective primary haemostasis because they lack or have an abnormal von Willebrand factor protein. Bleeding disorders are also seen in patients with a normally functioning blood clotting cascade and may be caused by a defective platelet function, thrombocytopenia, or even by unknown reasons. Furthermore, FVIIa may be used for treating excessive bleedings in other patients, such as, for example, bleedings in association with tissue damage, for example surgery or trauma, especially in tissues rich in tissue factor (TF). FVIIa may be used as well as when the bleeding is diffuse and poorly responding to current haemostatic techniques and therapies (such as, e.g. haemorrhagic gastritis and profuse uterine bleeding). FVIIa may also be suitable for the treatment of bleedings occurring in organs with limited possibility for mechanical haemostasis such as brain, inner ear region, eyes as well as in association with the process of taking biopsies from various organs and in laparoscopic surgery.

US 6310183 and US 6833352 relates to subcutaneous administration of FVIIa and FVIIa related polypeptides.

International Patent Application No. WO 93/07890 relates to the treatment of haemophilia with FIX by subcutaneous injection.

International Patent Application No. WO 95/26750 relates to a formulation of FVIII or FIX suitable for subcutaneous injection for treatment of haemophilia A or B.

International Patent Application No. WO 95/28954 relates to concentrated preparations of FIX suitable for storage and subcutaneous injection.

## BRIEF DESCRIPTION OF THE DRAWINGS

**Figure 1** shows the full amino acid sequence of native (wild type) human coagulation Factor VII (SEQ ID NO:1).

**Figure 2** illustrates the mean ($\pm$SD) of FVIIa plasma concentration determined via clot equivalent activity versus time profile for administration of 15 mg/ml rFVIIa in volumes of 200 $\mu$l, 500 $\mu$l or 1 ml.

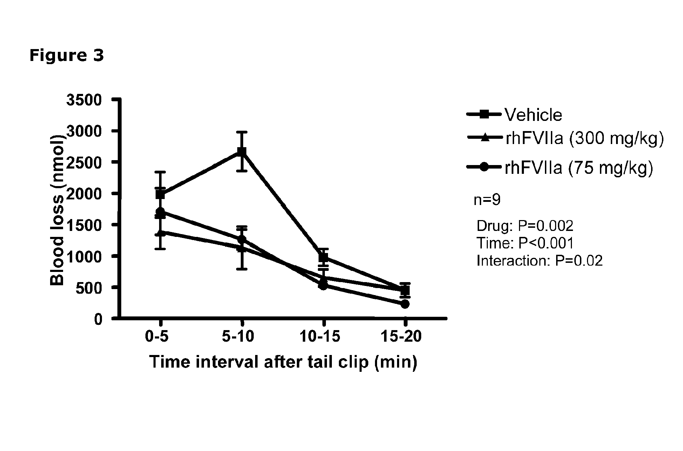

**Figure 3.** Effect on blood loss in different time periods after tail amputation in the sever tail clip model in haemophilia A mice. rhFVIIa refers to recombinant wild type human FVIIa.

## SUMMARY OF THE INVENTION

The present invention provides methods for treating a bleeding episode which are carried out by administering to a patient in need of such treatment an effective amount for such treatment of a Factor VIIa polypeptide via subcutaneous or intramuscular route of administration in a single, high dose of the Factor VIIa polypeptide in a small volume.

In one broad aspect the present invention relates to subcutaneous or intramuscular administration of FVIIa polypeptides.

In a frist aspect the present invention relates to the use of a FVIIa polypeptide for the preparation of a pharmaceutical formulation comprising the Factor VIIa polypeptide together with a pharmaceutically acceptable carrier for the on-demand treatment of a bleeding episode, wherein the pharmaceutical formulation is for subcutaneous or intramuscular administration in a single, high dose of said Factor VIIa polypeptide in a volume less than 1 ml.

In a second aspect the present invention relates to a method for treating an on-demand bleeding episode, the method comprising administering to a patient in need of such treatment an effective amount for such treatment of a Factor VIIa polypeptide, wherein the administering is via a subcutaneous or intramuscular route in a single, high dose of said Factor VIIa polypeptide in a volume less than 1 ml.

In some embodiments, the Factor VIIa polypeptide is wild type human FVIIa (NovoSeven®). In some embodiments, the Factor VIIa polypeptide is an amino acid sequence variant of Factor VIIa.

## DETAILED DESCRIPTION OF THE INVENTION

The present invention encompasses the subcutaneous or intramuscular administration of Factor VIIa polypeptides, such as wild-type Factor VIIa. It has now been found that Factor VIIa polypeptides can be effectively administered via a subcutaneous route in a single, high dose of the Factor VIIa polypeptide and in a small volume, providing therapeutically beneficial methods for treating Factor VIIa-responsive syndromes, such as, e.g., bleeding.

The problem to be solved may therefore been seen as an alternative way of administration, wherein a Factor VIIa polypeptide is administered in such a way as to effectively be able to treat an on demand bleeding.

This is solved by administering FVIIa subcutaneously in a volume less than 1 ml. The inventors of the present inventions have discovered, that such administration surprisingly gives higher bioavailability as well as faster absorption to the blood volume, compared to administration of volumes of 1 ml and above.

It has also been observed that subcutaneous administration of FVIIa can reduce the bleeding time in haemophilia A mice, in a similar fashion as intravenous injections.

The current treatment paradigm in therapy with FVIIa is i.v. administration of a single dose of 270 μg/kg. The bioavailability of FVIIa injected in volumes less than 1 ml in minipigs is about 50%. This translates to a therapeutical dose of 540 μg/kg with s.c. administration. FVIIa polypeptides injected in volumes less than 1 ml will also provide a simpler and less painful administration mode. Furthermore, the patient avoids the problems of repeated venous access, problems with finding veins in small children, infections in surgically inserted injection ports, destroyed peripheral veins etc.

The term "bleeding episodes" is meant to include uncontrolled and excessive bleeding which is a major problem both in connection with surgery and other forms of tissue damage. Uncontrolled and excessive bleeding may occur in subjects having a normal coagulation system and subjects having coagulation or bleeding disorders. Clotting factor deficiencies (haemophilia A and B, deficiency of coagulation factors XI or VII) or clotting factor inhibitors may be the cause of bleeding disorders. Excessive bleedings also occur in subjects with a normally functioning blood clotting cascade (no clotting factor deficiencies or -inhibitors against any of the coagulation factors) and may be caused by a defective platelet function, thrombocytopenia or von Willebrand's disease. In such cases, the bleedings may be likened to those bleedings caused by haemophilia because the haemostatic system, as in haemophilia, lacks or has abnormal essential clotting "compounds" (such as platelets or von Willebrand factor protein) that causes major bleedings. In subjects who experience extensive tissue damage in association with surgery or vast trauma, the normal haemostatic mechanism may be overwhelmed by the demand of immediate haemostasis and they may develop bleeding in spite of a normal haemostatic mechanism. Achieving satisfactory haemostasis also is a problem when bleedings occur in organs such as the brain, inner ear region and eyes with limited possibility for surgical haemostasis. The same problem may arise in the process of taking biopsies from various organs (liver, lung, tumour tissue, gastrointestinal tract) as well as in laparoscopic surgery. Common for all these situations is the difficulty to provide haemostasis by surgical techniques (sutures, clips, etc.) which also is the case when bleeding is diffuse (haemorrhagic gastritis and profuse uterine bleeding). Acute and profuse bleedings may also occur in subjects on anticoagulant therapy in whom a defective haemostasis has been induced by the therapy given. Such subjects may need surgical interventions in case the anticoagulant effect has to be counteracted rapidly. Radical retropubic prostatectomy is a commonly performed procedure for subjects with localized prostate cancer. The operation is frequently complicated by significant and sometimes massive blood loss. The considerable blood loss during prostatectomy is mainly related to the complicated anatomical situation, with various densely vascularised sites that are not easily accessible for surgical haemostasis, and which may result in diffuse bleeding from a large area. Another situation that may cause problems in the case of unsatisfactory haemostasis is when subjects with a normal haemostatic mechanism are given anticoagulant therapy to prevent thromboembolic disease. Such therapy may include heparin, other forms of proteoglycans, warfarin or other forms of vitamin K-antagonists as well as aspirin and other platelet aggregation inhibitors.

In one embodiment of the invention, the bleeding is associated with haemophilia. In one embodiment of the invention, the bleeding is associated with haemophilia A. In one embodiment of the invention, the bleeding is associated with haemophilia B. In another embodiment, the bleeding is associated with haemophilia with aquired inhibitors. In another embodiment, the bleeding is associated with thrombocytopenia. In another embodiment, the bleeding is associated with von Willebrand's disease. In another embodiment, the bleeding is associated with severe tissue damage. In another embodiment, the bleeding is associated with severe trauma. In another embodiment, the bleeding is associated with surgery. In another embodiment, the bleeding is associated with laparoscopic surgery. In another embodiment, the bleeding is associated with haemorrhagic gastritis. In another embodiment, the bleeding is profuse uterine bleeding. In another embodiment, the bleeding is occurring in organs with a limited possibility for mechanical haemostasis. In another embodiment, the bleeding is occurring in the brain, inner ear region or eyes. In another embodiment, the bleeding is associated with the process of taking biopsies. In another embodiment, the bleeding is associated with anticoagulant therapy.

The term "on-demand" as used herein refers to the treatment of an on-going bleeding episode, i.e. a bleeding that is already in progress, such as an acute bleeding episode that requires instant and immediate hemostatic treatment. Thus "on-demand" is distinguished from administering a hemostatic agent prior to the awareness or perception that bleeding has begun, such as, e.g., a prophylactic administration in advance of an anticipated bleeding situation.

The term "subject" as used herein is intended to mean any animal, in particular mammals, such as humans, and may, where appropriate, be used interchangeably with the term "patient".

Factor VIIa polypeptides

As used herein, the terms "Factor VIIa polypeptide" and "FVIIa polypeptide" means any protein in its pro-coagulant active form (Factor VII is cleaved between residues 152 and 153) comprising the amino acid sequence 1-406 of wild-type human Factor VIIa (i.e., a polypeptide having the amino acid sequence disclosed in U.S. Patent No. 4,784,950), variants thereof as well as Factor VII-related polypeptides, Factor VII derivatives and Factor VII conjugates. This includes Factor VII variants, Factor VII-related polypeptides, Factor VII derivatives and Factor VII conjugates exhibiting substantially the same or improved biological activity relative to wild-type human Factor VIIa.

The terms "Factor VII" or "FVII" are intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa. Such variants of Factor VII may exhibit different properties relative to human Factor VII, including stability, phospholipid binding, altered specific activity, and the like.

As used herein, "wild type human Factor VIIa" is a polypeptide having the amino acid sequence disclosed in U.S. Patent No. 4,784,950.

As used herein, "Factor VII-related polypeptides" encompasses polypeptides, including variants (or analogues), in which the Factor VIIa biological activity has been substantially modified, such as reduced, relative to the activity of wild-type Factor VIIa. These polypeptides include, without limitation, Factor VII or Factor VIIa into which specific amino acid sequence alterations have been introduced that modify or disrupt the bioactivity of the polypeptide.

The term "Factor VII derivative" as used herein, is intended to designate a Factor VII polypeptide exhibiting substantially the same or improved biological activity relative to wild-type Factor VII, in which one or more of the amino acids of the parent peptide have been genetically and/or chemically and/or enzymatically modified, e.g. by alkylation, glycosylation, PEGylation, GlycoPEGylation, acylation, ester formation or amide formation or the like. This includes but is not limited to PEGylated human Factor VIIa, cysteine-PEGylated human Factor VIIa and variants thereof. Non-limiting examples of Factor VII derivatives includes GlycoPegylated Factor VII derivatives as disclosed in WO 03/31464 and US Patent applications US 20040043446, US 20040063911, US 20040142856, US 20040137557, and US 20040132640 (Neose Technologies, Inc.); Factor VII conjugates as disclosed in WO 01/04287, US patent application 20030165996, WO 01/58935, WO 03/93465 (Maxygen ApS) and WO 02/02764, US patent application 20030211094 (University of Minnesota).

The term "improved biological activity" refers to Factor VII polypeptides with i) substantially the same or increased proteolytic activity compared to recombinant wild type human Factor VIIa or ii) to Factor VII polypeptides with substantially the same or increased TF binding activity compared to recombinant wild type human Factor VIIa or iii) to Factor VII polypeptides with substantially the same or increased half life in blood plasma compared to recombinant wild type human Factor VIIa. The term "PEGylated human Factor VIIa" means human Factor VIIa, having a PEG molecule conjugated to a human Factor VIIa polypeptide. It is to be understood, that the PEG molecule may be attached to any part of the Factor VIIa polypeptide including any amino acid residue or carbohydrate moiety of the Factor VIIa polypeptide. The term "cysteine-PEGylated human Factor VIIa" means Factor VIIa having a PEG molecule conjugated to a sulfhydryl group of a cysteine introduced in human Factor VIIa.

Non-limiting examples of Factor VII variants having substantially the same or increased proteolytic activity compared to recombinant wild type human Factor VIIa include S52A- Factor VIIa, S60A-Factor VIIa (Lino et al., Arch. Biochem. Biophys. 352: 182-192, 1998); Factor VIIa variants exhibiting increased proteolytic stability as disclosed in U.S. Patent No. 5,580,560; Factor VIIa that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); Factor VII variants as disclosed in PCT/DK02/00189 (corresponding to WO 02/077218); and Factor VII variants exhibiting increased proteolytic stability as disclosed in WO 02/38162 (Scripps Research Institute); Factor VII variants having a modified Gla-domain and exhibiting an enhanced membrane binding as disclosed in WO 99/20767, US patents US 6017882 and US 6747003, US patent application 20030100506 (University of Minnesota) and WO 00/66753, US patent applications US 20010018414, US 2004220106, and US 200131005, US patents US 6762286 and US 6693075 (University of Minnesota); and Factor VII variants as disclosed in WO 01/58935, US patent US 6806063, US patent application 20030096338 (Maxygen ApS), WO 03/93465 (Maxygen ApS) and WO 04/029091 (Maxygen ApS).

Non-limiting examples of Factor VII variants having increased biological activity compared to wild-type Factor VIIa include Factor VII variants as disclosed in WO 01/83725, WO 02/22776, WO 02/077218, PCT/DK02/00635 (corresponding to WO 03/027147), Danish patent application PA 2002 01423 (corresponding to WO 04/029090), Danish patent application PA 2001 01627 (corresponding to WO 03/027147); WO 02/38162 (Scripps Research Institute); and Factor VIIa variants with enhanced activity as disclosed in JP 2001061479 (Chemo-Sero-Therapeutic Res Inst.).

Examples of variants of factor VII include, without limitation, P10Q-FVII, K32E-FVII, P10Q/K32E-FVII, L305V-FVII, L305V/M306D/D309S-FVII, L305I-FVII, L305T-FVII, F374P-FVII, V158T/M298Q-FVII, V158D/E296V/M298Q-FVII, K337A-FVII, M298Q-FVII, V158D/M298Q-FVII, L305V/K337A-FVII, V158D/E296V/M298Q/L305V-FVII, V158D/E296V/M298Q/K337A-FVII, V158D/E296V/M298Q/L305V/K337A-FVII, K157A-FVII, E296V-FVII, E296V/M298Q-FVII, V158D/E296V-FVII, V158D/M298K-FVII, and S336G-FVII, L305V/K337A-FVII, L305V/V158D-FVII, L305V/E296V-FVII, L305V/M298Q-FVII, L305V/V158T-FVII, L305V/K337A/V158T-FVII, L305V/K337A/M298Q-FVII, L305V/K337A/E296V-FVII, L305V/K337A/V158D-FVII, L305V/V158D/M298Q-FVII, L305V/V158D/E296V-FVII, L305V/V158T/M298Q-FVII, L305V/V158T/E296V-FVII, L305V/E296V/M298Q-FVII, L305V/V158D/E296V/M298Q-FVII, L305V/V158T/E296V/M298Q-FVII, L305V/V158T/K337A/M298Q-FVII, L305V/V158T/E296V/K337A-FVII, L305V/V158D/K337A/M298Q-FVII, L305V/V158D/E296V/K337A-FVII, L305V/V158D/E296V/M298Q/K337A-FVII, L305V/V158T/E296V/M298Q/K337A-FVII, S314E/K316H-FVII, S314E/K316Q-FVII, S314E/L305V-FVII, S314E/K337A-FVII, S314E/V158D-FVII, S314E/E296V-FVII, S314E/M298Q-FVII, S314E/V158T-FVII, K316H/L305V-FVII, K316H/K337A-FVII, K316H/V158D-FVII, K316H/E296V-FVII, K316H/M298Q-FVII, K316H/V158T-FVII, K316Q/L305V-FVII, K316Q/K337A-FVII, K316Q/V158D-FVII, K316Q/E296V-FVII, K316Q/M298Q-FVII, K316Q/V158T-FVII, S314E/L305V/K337A-FVII, S314E/L305V/V158D-FVII, S314E/L305V/E296V-FVII, S314E/L305V/M298Q-FVII, S314E/L305V/V158T-FVII, S314E/ L305V/K337A/V158T-FVII, S314E/L305V/K337A/M298Q-FVII, S314E/L305V/K337A/E296V-FVII, S314E/L305V/ K337A/V158D-FVII, S314E/L305V/V158D/M298Q-FVII, S314E/L305V/V158D/E296V-FVII, S314E/L305V/V158T/ M298Q-FVII, S314E/L305V/V158T/E296V-FVII, S314E/L305V/E296V/M298Q-FVII, S314E/L305V/V158D/E296V/ M298Q-FVII, S314E/L305V/V158T/E296V/M298Q-FVII, S314E/L305V/V158T/K337A/M298Q-FVII, S314E/L305V/ V158T/E296V/K337A-FVII, S314E/L305V/V158D/K337A/M298Q-FVII, S314E/L305V/V158D/E296V/K337A-FVII, S314E/L305V/V158D/E296V/M298Q/K337A-FVII, S314E/L305V/V158T/E296V/M298Q/K337A-FVII, K316H/L305V/ K337A-FVII, K316H/L305V/V158D-FVII, K316H/L305V/E296V-FVII, K316H/L305V/M298Q-FVII, K316H/L305V/V158T-FVII, K316H/L305V/K337A/V158T-FVII, K316H/L305V/K337A/M298Q-FVII, K316H/L305V/K337A/E296V-FVII, K316H/ L305V/K337A/V158D-FVII, K316H/L305V/V158D/M298Q-FVII, K316H/L305V/V158D/E296V-FVII, K316H/L305V/ V158T/M298Q-FVII, K316H/L305V/V158T/E296V-FVII, K316H/L305V/E296V/M298Q-FVII, K316H/L305V/V158D/ E296V/M298Q-FVII, K316H/L305V/V158T/E296V/M298Q-FVII, K316H/L305V/V158T/K337A/M298Q-FVII, K316H/ L305V/V158T/E296V/K337A-FVII, K316H/L305V/V158D/K337A/M298Q-FVII, K316H/L305V/V158D/E296V/K337A -FVII, K316H/L305V/V158D/E296V/M298Q/K337A-FVII, K316H/L305V/V158T/E296V/M298Q/K337A-FVII, K316Q/ L305V/K337A-FVII, K316Q/L305V/V158D-FVII, K316Q/L305V/E296V-FVII, K316Q/L305V/M298Q-FVII, K316Q/ L305V/V158T-FVII, K316Q/L305V/K337A/V158T-FVII, K316Q/L305V/K337A/M298Q-FVII, K316Q/L305V/K337A/ E296V-FVII, K316Q/L305V/K337A/V158D-FVII, K316Q/L305V/V158D/M298Q-FVII, K316Q/L305V/V158D/E296V-FVII, K316Q/L305V/V158T/M298Q-FVII, K316Q/L305V/V158T/E296V-FVII, K316Q/L305V/E296V/M298Q-FVII, K316Q/L305V/V158D/E296V/M298Q-FVII, K316Q/L305V/V158T/E296V/M298Q-FVII, K316Q/L305V/V158T/K337A/ M298Q-FVII, K316Q/L305V/V158T/E296V/K337A-FVII, K316Q/L305V/V158D/K337A/M298Q-FVII, K316Q/L305V/ V158D/E296V/K337A -FVII, K316Q/L305V/V158D/E296V/M298Q/K337A-FVII, K316Q/L305V/V158T/E296V/M298Q/ K337A-FVII, F374Y/K337A-FVII, F374Y/V158D-FVII, F374Y/E296V-FVII, F374Y/M298Q-FVII, F374Y/V158T-FVII, F374Y/S314E-FVII, F374Y/L305V-FVII, F374Y/L305V/K337A-FVII, F374Y/L305V/V158D-FVII, F374Y/L305V/E296V-FVII, F374Y/L305V/M298Q-FVII, F374Y/L305V/V158T-FVII, F374Y/L305V/S314E-FVII, F374Y/K337A/S314E-FVII, F374Y/K337A/V158T-FVII, F374Y/K337A/M298Q-FVII, F374Y/K337A/E296V-FVII, F374Y/K337A/V158D-FVII, F374Y/ V158D/S314E-FVII, F374Y/V158D/M298Q-FVII, F374Y/V158D/E296V-FVII, F374Y/V158T/S314E-FVII, F374Y/ V158T/M298Q-FVII, F374Y/V158T/E296V-FVII, F374Y/E296V/S314E-FVII, F374Y/S314E/M298Q-FVII, F374Y/ E296V/M298Q-FVII, F374Y/L305V/K337A/V158D-FVII, F374Y/L305V/K337A/E296V-FVII, F374Y/L305V/K337A/ M298Q-FVII, F374Y/L305V/K337A/V158T-FVII, F374Y/L305V/K337A/S314E-FVII, F374Y/L305V/V158D/E296V-FVII, F374Y/L305V/V158D/M298Q-FVII, F374Y/L305V/V158D/S314E-FVII, F374Y/L305V/E296V/M298Q-FVII, F374Y/ L305V/E296V/V158T-FVII, F374Y/L305V/E296V/S314E-FVII, F374Y/L305V/M298Q/V158T-FVII, F374Y/L305V/ M298Q/S314E-FVII, F374Y/L305V/V158T/S314E-FVII, F374Y/K337A/S314E/V158T-FVII, F374Y/K337A/S314E/ M298Q-FVII, F374Y/K337A/S314E/E296V-FVII, F374Y/K337A/S314E/V158D-FVII, F374Y/K337A/V158T/M298Q-FVII, F374Y/K337A/V158T/E296V-FVII, F374Y/K337A/M298Q/E296V-FVII, F374Y/K337A/M298Q/V158D-FVII, F374Y/K337A/E296V/V158D-FVII, F374Y/V158D/S314E/M298Q-FVII, F374Y/V158D/S314E/E296V-FVII, F374Y/ V158D/M298Q/E296V-FVII, F374Y/V158T/S314E/E296V-FVII, F374Y/V158T/S314E/M298Q-FVII, F374Y/V158T/ M298Q/E296V-FVII, F374Y/E296V/S314E/M298Q-FVII, F374Y/L305V/M298Q/K337A/S314E-FVII, F374Y/L305V/ E296V/K337A/S314E-FVII, F374Y/E296V/M298Q/K337A/S314E-FVII, F374Y/L305V/E296V/M298Q/K337A -FVII, F374Y/L305V/E296V/M298Q/S314E-FVII, F374Y/V158D/E296V/M298Q/K337A-FVII, F374Y/V158D/E296V/M298Q/ S314E-FVII, F374Y/L305V/V158D/K337A/S314E-FVII, F374Y/V158D/M298Q/K337A/S314E-FVII, F374Y/V158D/ E296V/K337A/S314E-FVII, F374Y/L305V/V158D/E296V/M298Q-FVII, F374Y/L305V/V158D/M298Q/K337A-FVII, F374Y/L305V/V158D/E296V/K337A-FVII, F374Y/L305V/V158D/M298Q/S314E-FVII, F374Y/L305V/V158D/ E296V/S314E-FVII, F374Y/V158T/E296V/M298Q/K337A-FVII, F374Y/V158T/E296V/M298Q/S314E-FVII, F374Y/ L305V/V158T/K337A/S314E-FVII, F374Y/V158T/M298Q/K337A/S314E-FVII, F374Y/V158T/E296V/K337A/S314E-FVII, F374Y/L305V/V158T/E296V/M298Q-FVII, F374Y/L305V/V158T/M298Q/K337A-FVII, F374Y/L305V/V158T/ E296V/K337A-FVII, F374Y/L305V/V158T/M298Q/S314E-FVII, F374Y/L305V/V158T/E296V/S314E-FVII, F374Y/ E296V/M298Q/K337A/V158T/S314E-FVII, F374Y/V158D/E296V/M298Q/K337A/S314E-FVII, F374Y/L305V/V158D/ E296V/M298Q/S314E-FVII, F374Y/L305V/E296V/M298Q/V158T/S314E-FVII, F374Y/L305V/E296V/M298Q/K337A/ V158T-FVII, F374Y/L305V/E296V/K337A/V 158T/S314E-FVII, F374Y/L305V/M298Q/K337A/V158T/S314E-FVII, F374Y/L305V/V158D/E296V/M298Q/K337A-FVII, F374Y/L305V/V158D/E296V/K337A/S314E-FVII, F374Y/L305V/ V158D/M298Q/K337A/S314E-FVII, F374Y/L305V/E296V/M298Q/K337A/V158T/S314E-FVII, F374Y/L305V/V158D/ E296V/M298Q/K337A/S314E-FVII, S52A-Factor VII, S60A-Factor VII; R152E-Factor VII, S344A-Factor VII, T106N-FVII, K143N/N145T-FVII, V253N-FVII, R290N/A292T-FVII, G291N-FVII, R315N/V317T-FVII, K143N/N145T/ R315N/V317T-FVII; and FVII having substitutions, additions or deletions in the amino acid sequence from 233Thr to 240Asn; FVII having substitutions, additions or deletions in the amino acid sequence from 304Arg to 329Cys; and FVII having substitutions, additions or deletions in the amino acid sequence from 153Ile to 223Arg.

Thus, substitution variants in a factor VII polypeptide include, without limitation substitutions in positions P10, K32, L305, M306, D309, L305, L305, F374, V158, M298, V158, E296, K337, M298, M298, S336, S314, K316, K316, F374, S52, S60, R152, S344, T106, K143, N145, V253, R290, A292, G291, R315, V317, and substitutions, additions or deletions in the amino acid sequence from T233 to N240 or from R304 to C329; or from I153 to R223, or combinations thereof, in particular variants such as P10Q, K32E, L305V, M306D, D309S, L305I, L305T, F374P, V158T, M298Q, V158D, E296V, K337A, M298Q, M298K, S336G, S314E, K316H, K316Q, F374Y, S52A, S60A, R152E, S344A, T106N, K143N, N145T, V253N, R290N, A292T, G291N, R315N, V317T, and substitutions, additions or deletions in the amino acid sequence from T233 to N240, or from R304 to C329, or from I153 to R223, or combinations thereof.

**Pharmacokinetic Variants**

In one series of embodiments, Factor VIIa polypeptides for use in the present invention encompass those for which one or more pharmacokinetic properties has been altered relative to wild type Factor VIIa.

In practicing the present invention, pharmacokinetic properties may be calculated using, e.g., WinNonlin Professional Version 3.1 (Pharsight Inc., Mountain View, CA, USA). Calculations are performed using mean concentration values at each time point, if more than one value was present.

The following pharmacokinetic parameters may be calculated: AUC, $AUC_{\%Extrap}$, $C_{max}$, $t_{max}$, $\lambda_z$, $t_{1/2}$, CL, and $V_z$ using the following formulas:

AUC       Area under the plasma concentration-time curve from time 0 to infinity. Calculated using the linear/log trapezoidal rule with extrapolation to infinity.

The linear trapezoidal rule is used from time 0 to $t_{max}$:

$$AUC(0 - t_{max}) = \left( \sum_{i=1}^{n-1} \frac{C(i) + C(i+1)}{2} \cdot (t(i+1) - t(i)) \right)$$

The log trapezoidal rule is used from time $t_{max}$ to the last time point t:

$$AUC(t_{max} - t) = \left( \sum_{i=1}^{n-1} \frac{C(i) - C(i+1)}{\ln\left(\frac{C(i)}{C(i+1)}\right)} \cdot (t(i+1) - t(i)) \right)$$

Extrapolation to infinity is performed using:

$$AUC(t - \infty) = \frac{C(t)}{\lambda_z}$$

$AUC_{\%Extrap}$    Percentage of AUC that is due to extrapolation from the last concentration to infinity:

$$AUC_{\%Extrap} = \frac{AUC(t - \infty)}{AUC} \cdot 100\%$$

$C_{max}$       Maximum plasma concentration back extrapolated to time zero
CL        Total body clearance

$$CL = \frac{Dose}{AUC}$$

| $t_{max}$ | Time at which maximum plasma concentration is observed. |
| $t_{1/2}$ | Half-life: |

$$t_{\frac{1}{2}} = \frac{\ln 2}{\lambda_z}$$

| $\lambda_z$ | Terminal rate constant. Calculated by log-linear regression of (mean) concentrations versus time |
| $V_z$ | Volume of distribution based on the terminal phase: |

$$V_z = \frac{Dose}{AUC \cdot \lambda_z}$$

Non-limiting examples of useful Factor VIIa polypeptides include those in which the ratio between absorption and clearance has been altered to provide increased AUC and/or $t_{1/2}$.

In some embodiments, Factor VIIa polypeptides are those in which the modification alters the ratio between adsorption and clearance resulting in an increase in bioavailability of at least about 25%, 50%, 75%, 100%, 125%, 150%, 200%, or 500% of the bioavailability of wild-type Factor VII.

Preparation of compound

Human purified Factor VIIa suitable for use in the present invention is preferably made by DNA recombinant technology, e.g. as described by Hagen et al., Proc.Natl.Acad.Sci. USA 83: 2412-2416, 1986 or as described in European Patent No. 200.421 (ZymoGenetics).

The Factor VII variants described herein may be produced by means of recombinant nucleic acid techniques. In general, a cloned wild-type Factor VII nucleic acid sequence is modified to encode the desired protein. This modified sequence is then inserted into an expression vector, which is in turn transformed or transfected into host cells. Higher eukaryotic cells, in particular cultured mammalian cells, are preferred as host cells. The complete nucleotide and amino acid sequences for human Factor VII are known (see U.S. 4,784,950, where the cloning and expression of recombinant human Factor VII is described). The bovine Factor VII sequence is described in Takeya et al., J. Biol. Chem. 263: 14868-14872 (1988)). It will be understood that any conventional technique may be used to produce Factor VIIa sequence variants.

Factor VII may also be produced by the methods described by Broze and Majerus, J. Biol.Chem. 255 (4): 1242-1247, 1980 and Hedner and Kisiel, J. Clin.Invest. 71: 1836-1841, 1983. These methods yield Factor VII without detectable amounts of other blood coagulation Factors. An even further purified Factor VII preparation may be obtained by including an additional gel filtration as the final purification step.

Chemical and/or enzymatic modification of wild-type Factor VIIa or Factor VIIa sequence variants may be achieved by any means known in the art. Suitable modifications include, without limitation, chemical glycan modifications as described in EP application, EP06120000 (Novo Nordisk A/S), C-terminal modifications as described in WO2006013202, glycosyltransferase mediated modification as described in WO2006035057, dendrimer modification as described in WO2005014049, carboxypeptidase mediated terminal modification (as described, e.g., in WO2005035553-A2, WO9520039-A, and WO9838285-A), transglutaminase-mediated modification (as described, e.g., WO2005070468), autocatalytic/endopeptidase-mediated transpeptidation (as described, e.g., in WO2006013202-A2); thiol-mediated modification (as described, e.g., in WO2002077218 and PCT/EP/2006063310) and amine-mediated modification (as described, e.g., in WO02/02764.

Factor VII and Factor VII polypeptides may be activated by proteolytic cleavage, using Factor XIIa or other proteases having trypsin-like specificity, such as, e.g., Factor IXa, kallikrein, Factor Xa, and thrombin. See, e.g., Osterud et al., Biochem. 11:2853 (1972); Thomas, U.S. Patent No. 4,456,591; and Hedner et al., J. Clin. Invest. 71:1836 (1983). Alternatively, Factor VII may be activated by passing it through an ion-exchange chromatography column, such as Mono Q® (Pharmacia) or the like. The resulting activated Factor VII may then be formulated and administered as described below.

<u>Pharmaceutical administration</u>

The regimen for any patient to be treated with FVIIa as mentioned herein should be determined by those skilled in the art. The dose to be administered in therapy may depend on numerous factors, such as, e.g., the weight and the condition of the patient, and can be determined by examining different points in a matrix of treatment and correlating to clinical outcomes.

In one series of embodiments, a Factor-VIIa related polypeptide is administered subcutaneously and in an amount of about 100-100,000 units per kg body weight, such as, e.g., in an amount of about 250 - 25,000 units per kg body weight corresponding to about 5-500 $\mu$g/kg.

In practicing the present invention, Factor VIIa polypeptide may be administered to a subject as a single dose comprising a single-dose-effective amount for treating the bleeding, or in a staged series of doses which together comprise an effective amount for treating the bleeding. An effective amount of the Factor VIIa polypeptide refers to the amount of Factor VIIa polypeptide which, when administered in a single dose or in the aggregate of multiple doses, or as part of any other type of defined treatment regimen, produces a measurable improvement in at least one clinical parameter associated with the bleeding. When Factor VIIa polypeptides with different activity are administered, an effective amount may be determined by comparing one or more biological properties (including e.g, the coagulant activity and/or one or more pharmacokinetic properties) of the Factor VIIa polypeptides with that of known Factor VIIa polypeptides and adjusting the amount to be administered proportionately to the predetermined effective dose for the known Factor VIIa polypeptides.

Administration of a single dose refers to administration of an entire dose of Factor VIIa polypeptide as a bolus over a period of less than about 5 minutes. In some embodiments, the administration occurs over a period of less than about 2.5 minutes, and, in some, over less than about 1 min. Typically, a single-dose effective amount comprises at least about 40 $\mu$g/kg human Factor VIIa polypeptide compared to wild-type Factor VIIa, such as, at least about 50 $\mu$g/kg, 75 $\mu$g/kg, or 90 $\mu$g/kg, or at least 150 $\mu$g/kg Factor VIIa. This dosis may vary depending on activity of the FVIIa polypeptide as well as the specific indication being treated.

In some embodiments, following administration of a single dose of Factor VIIa polypeptide, the subject receives no further Factor VIIa polypeptide for an interval of at least about 30 minutes. In some embodiments the post-administration interval is at least about 45 minutes, such as at least about 1 hour, at least about 1.5 hours, or at least about 2 hours.

In other embodiments, the subject receives Factor VIIa polypeptide according to the following regimen: (i) The subject receives a first amount of Factor VIIa polypeptide comprising at least about 40 $\mu$g/kg; (ii) after a period of at least about 30 minutes, a second amount of Factor VIIa polypeptide is administered, the amount comprising at least about 40 $\mu$g/kg; and (iii) after a period of at least about 30 minutes from administration of the second dose, a third amount of Factor VIIa polypeptide is administered, the amount comprising at least about 40 $\mu$g/kg. After a period of at least about 30 minutes following the administration of the third amount, the subject may then receive a further (fourth) amount of Factor VIIa polypeptide comprising at least about 40 $\mu$g/kg.

In other embodiments, the first amount of Factor VIIa polypeptide comprises at least about 40 $\mu$g/kg, such as at least about 80 $\mu$g/kg, such as at least about 100 $\mu$g/kg or at least about 150 $\mu$g/kg or at least 200 $\mu$g/kg or at least 300 $\mu$g/kg or at least 500 $\mu$g/kg; in other embodiments, the second amount of Factor VIIa polypeptide comprises at least about 75 $\mu$g/kg, such as at least about 90 $\mu$g/kg; in other embodiments, the third (and optionally fourth) amount of a Factor VIIa polypeptide comprises at least about 75 $\mu$g/kg, such as at least about 90 $\mu$g/kg.

In one embodiment, the first dose comprises about 200 $\mu$g/kg, the second dose about 100 $\mu$g/kg, and the third (and optionally fourth) dose about 100 $\mu$g/kg.

In other embodiments, the subject receives the second amount of a Factor VIIa polypeptide after a period of at least about 45 minutes from the first administration, such as at least about 1 hour, at least about 1.5 hours, at least about 2 hours, at least about 2.5 hours, or at least about 3 hours.

In one embodiment, the subject receives a first dose comprising about 200 $\mu$g/kg; after a period of about 1 hour, the subject receives a second dose comprising about 100 $\mu$g/kg, and after a period of about 3 hours from the first dose, the subject receives a third dose comprising about 100 $\mu$g/kg.

*Combination treatments:*

The present invention encompasses combined administration of an additional agent in concert with a Factor VIIa polypeptide. In some embodiments, the additional agent comprises a coagulant, including, without limitation, a coagulation factor such as, e.g., Factor VIII, Factor IX, Factor V, Factor XI, or Factor XIII; or an inhibitor of the fibrinolytic system, such as, e.g., PAI-1, aprotinin, $\epsilon$-aminocaproic acid or tranexamic acid.

It will be understood that, in embodiments comprising administration of combinations of Factor VIIa polypeptides with other agents, the dosage of the Factor VIIa polypeptide may on its own comprise an effective amount and additional agent(s) may further augment the therapeutic benefit to the patient. Alternatively, the combination of Factor VIIa polypeptide and the second agent may together comprise an effective amount. It will also be understood that effective amounts may be defined in the context of particular treatment regimens, including, e.g., timing and number of administrations, modes of administrations, formulations, etc.

Formulation of medicament

An intravenous injection is normally 5-20 ml. According to the present invention the injection given subcutaneously should be below 1 ml. The concentration of the FVIIa-related must therefore be relatively high in such a medicament.

The volume administered can be more than 0.01 ml, such as more than 0.1 ml, such as more than 1.5 ml, such as more than 0.5 ml.

Additives increasing the bioavailability of the FVIIa-related polypeptide are suitably organic compounds *per se,* salts thereof, emulsions or dispersions containing organic compounds *per se* or salts thereof, e.g. dispersions of polar lipids, or any combination or sequence of addition thereof. Organic compounds useful in the invention are e.g. amino acids, peptides, proteins, and polysaccharides. Peptides include dipeptides, tripeptides, oligopeptides, such as collagen and gelatine. The collagen and gelatine is preferably hydrolysed. Polysaccharides include e.g. chitosans, cyclodextrins, starch, hyaluronic acids, dextrans, cellulose, and any derivatives, combinations and/or sequence of addition thereof. The starch is preferably hydrolysed. The emulsions include oil-in-water emulsions with oil as the dispersed phase and water-in-oil dispersions with oil as the continuous phase. The oil can be of vegetable or of animal origin or synthetically produced. Suitably, the vegetable oil of the emulsions is soybean oil or safflower oil, or any combination thereof. Suitably the polar liquids are one or more phospho- lipids or glycolipids or any combination thereof. The additives increasing the bioavailability of FVIIa could be added to the formulation before drying or upon reconstitution, or can be added to a stable solution or dispersion containing FVIIa.

Before administration, one or more aqueous solutions or dispersions can be added, in any mixture or sequence, to the medicament of the present invention, which is a stable aqueous solution, a dispersion or in dried form.

The medicament can be in a dried form, preferably freeze-dried. Before administration, the dried product or composition can be reconstituted with an aqueous solution or a dispersion e.g. a suspension, a liposomal formulation or an emulsion. The medicament can also be a stable aqueous solution ready for administration. It can also be a dispersion, e.g. a suspension, a liposomal formulation or an emulsion.

The FVIIa activity in the formulation is preferably from about 0.1 mg/ml to about 100 mg/ml, such as, e.g., from about 0.3 mg/ml to about 25 mg/ml, from about 0.6 mg/ml to about 25 mg/ml, from about 3 mg/ml to about 25 mg/ml or from about 6 mg/ml to about 25 mg/ml.

The medicament may also comprise one or more salts in order to give an isotonic solution, e.g. NaCl, KCl, and/or it may comprise one or more other isotonicity establishing compounds, preferably in an amount of more than 1.0 mg/ml.

Calcium, or other divalent metal ions, e.g. zinc, may be used as necessary for the maintenance of the FVIIa activity. It may be added as, for example, calcium chloride, but other salts such as calcium gluconate, calcium glubionate or calcium gluceptate may also be used. The medicament preferable comprises calcium chloride in an amount of more than 0.15 mg/ml.

An amino acid is preferably used to buffer the system and it also protects the protein if the formulation is freeze-dried. A suitable buffer can be glycine, lysine, arginine, histidine or glycylglycine, preferred is histidine.

A non-ionic surfactant may also be present in the medicament. The surfactant is preferable chosen from block-copolymers, such as a poloxamer, e.g. poloxamer 188, or a polyoxyethylene sorbitan fatty acid ester, such as polyoxyethylene-(20)-sorbitan monolaurate or polyoxyethylene-(80)-sorbitan monooleate. Preferred are polyoxyethylene-(80)-sorbitan monooleate (Tween 80). Tween 80 is preferably used in a concentration of at least 0.01 mg/ml. The non-ionic surfactant, if used, should preferably be present in an amount above the critical micelle concentration (CMC). See Wan and Lee, Journal of Pharm Sci, 63, p. 136, 1974.

Mono- or disaccharides (e.g. sucrose), polysaccharides such as low molecular weight dextrins, or sugar alcohols (e.g. sorbitol, glycerol or mannitol) may be added. The medicament may also comprise antioxidants such as bisulfite, ascorbate gluthathione, acetylcystein, tocopherol, methionin, EDTA, citric acid, butyl hydroxy toluene and /or butyl hydroxy anisole. Complexing agents, such as EDTA and citric acid can also be present in small concentrations for stabilising the FVIIa molecules, if they exhibit a stronger affinity for destabilising metal ions than for calcium or other divalent metal ions, e.g. zn2+. The medicament may also contain cyclodextrins, in particular sulfoalkyl ether cyclodextrins (WO2005023308). Furthermore, preservatives such as benzyl alcohol, phenol, sorbic acid, parabens, m-cresol and chlorocresol may be added.

The adjuvants are generally present in a concentration of from 0.001 to 4% w/v. The pharmaceutical preparation may also contain protease inhibitors, e.g. aprotinin or tranexamic acid.

The pH of the preparation is preferably adjusted to a value in the interval of 2 - 9. Preparations having a pH from about 5.0 to about 7.5 are preferred, more preferred are preparations having a pH from about 5.0 to about 6.5, most preferred are preparations having a pH from about 5.5 to about 6.0.

Preferably, the FVIIa-related polypeptide is highly purified, i.e. has a specific activity of more than 40 IU/μg.

In one embodiment, the medicament consists of

| | |
|---|---|
| rFVIIa-related polypeptide | 4 mg/ml (30,000 IU/ml) |
| Sodium chloride | 5.84 mg/ml |
| Polysorbate 80 | 0.1 mg/ml |
| Calcium chloride, 2H2O | 1.47 mg/ml |
| Histidine | 1.37 mg/ml |
| pH 6.0 | |

Conventional techniques for preparing pharmaceutical compositions, which can be used according to the present invention, are, for example, described in Remington: The Science and Practice of Pharmacy, 19th ed., 1995.

The medicaments may be sterilised by, for example, filtration through a bacteria-retaining filter, by incorporating sterilising agents into the medicaments, by irradiating the medicaments, or by heating the medicaments. They can also be manufactured in the form of sterile solid medicaments which can be dissolved in sterile water, or some other sterile injectable medium prior to or immediately before use.

The present invention is further illustrated by the following examples. The presented examples are meant as an illustration of the invention, not as a limitation.

## EXAMPLES

### Example 1

Pharmacokinetics of rFVIIa after s.c. administration in minipigs

Animals:

The study was performed in 16 female crossbred LYD pigs weighing approximately 25-30 kg from Gundsøgård, Gundsøgårdvej 1, Roskilde, Denmark. The study was performed in a thermostated room at 15-24 °C with a 12 h cycle of light and darkness. Light was on from 06.00 to 18.00 h. The animals were housed in pens with straw as bedding, one in each pen.

Drugs and chemicals:

The test substances were prepared by reconstituting a freeze dried substance in sterile water. Each vial contained 22.5 mg rFVIIa and was added 1.5 ml sterile water. After reconstitution, the vials contained rFVIIa (15 mg/ml), NaCl (0.39 mg/ml), $CaCl_2,2H_2O$ (5.15 mg/ml), Histidin (1.55 mg/ml), Methionin (0.5 mg/ml), Tween80 (0.07 mg/ml), Mannitol (25 mg/ml), Sucrose (10 mg/ml), pH 5.5.

Analytical methods:

The concentration of FVIIa was determined by an ELISA and the activity by a clot assay. The concentration of rFVIIa and analogues were determined by an ELISA and the activity by a clot assay.

ELISA:

The assay was basically the same as the Factor FVII EIA Kit from DakoCytomation, but modified to have higher sensitivity and to measure recombinant human FVIIa (rhFVIIa) in pig plasma. Briefly, rhFVIIa concentrations in diluted plasma samples were determined from calibrator rows of rhFVIIa prepared in SPA-buffer supplemented with pig plasma to the same percentage as in the samples. The ELISA set-up was a direct two-site sandwich based on two anti-rhFVIIa monoclonal antibodies, one serving to capture rhFVIIa and the other to detect bound rhFVIIa. Biotinylated detector antibody was incubated with diluted plasma sample or calibrator in 96-well microtiter plates coated with the capture antibody. Following, the wells were washed and incubated with horseradish peroxidase-labelled streptavidin, washed again, and incubated with a ready-to-use TMB/peroxide peroxidase colour substrate. Finally, the colour development was stopped by addition of $H_3PO_4$ and the optical density was measured at 450 nm and at 620 nm as reference.

Clot assay:

The FVIIa activity level of FVIIa polypeptides was analyzed using a modified FVIIa-clot assay as according to Morrisey et al.

In short, the activity of the FVIIa polypeptide was measured in a one-stage clotting assay employing the extracellular domains of tissue factor (soluble TF, sTF) which possesses cofactor activity for FVIIa but fails to support activation of FVII by FXa or FIXa[i]. sTF (TF 1-209, LOd 11338-125), test samples, and calibrator were diluted in 50 mM tris, 100 mM NaCl, 1% BSA, pH 7.4 (TBS/BSA) buffer. The calibrator (rFVIIa, bulk LASA 13200-008 - 1.31 mg/ml, 32 U/ug) was diluted to range from 2 - 200 pM and the calibrator was supplemented with mouse plasma to the same percentage as in the samples. Equal volumes (25 µl) of diluted samples (or calibrator) and congenital FVII deficient plasma (Helena Biosciences) were mixed. Rabbit brain cephalin (RBC, Heptest Laboratories Inc, one vial reconstituted in 10 ml 0.9 % NaCl) (50 ul) was added to rFVIIa sample/deficient plasma and allowed to incubate for 2 min at 37°C. Coagulation was initiated by addition of 50 ul sTF/$CaCl_2$ (sTF, 29 nM; $CaCl_2$, 4.2 mM). Clot times were recorded and plotted versus the concentration of rFVIIa on log-log scales and a linear regression curve was fitted to the log-transformed data by regression analysis. Clot times for the long-acting variants were converted to rFVIIa-like concentrations (nM) based on this calibration curve.

Analysis of results:

Results from clot activity analysis were subjected to non-compartmental pharmacokinetic analysis using the PC based software WinNonlin (Phar-sight Corporation).

Results and discussion:

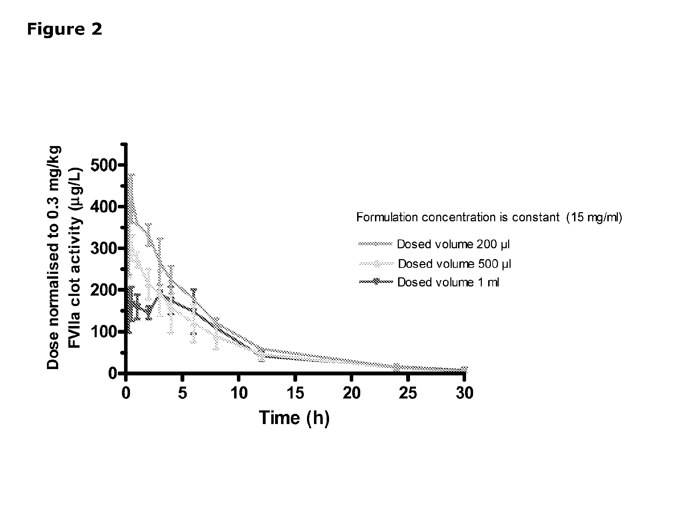

Figure 2 illustrates the mean ($\pm$SD) of FVIIa plasma concentration determined via clot equivalent activity versus time profile for administration of 15 mg/ml rFVIIa in volumes of 200 µl, 500 µl or 1 ml. The plasma concentrations have been normalized by the dose, correspond to a dose level of 0,3 mg/kg. It is clear that animals administered a dose volume below 1 ml experience a higher plasma concentration immediately after administration, and a larger area under the curve.

## Example 2

Tail bleeding in haemophilia A mice after subcutaneous administration of activated Factor VIIa.

Method

*Animals*

Twenty seven VIII knockout mice were obtained from the colony at Taconic M&B (Denmark). The animals were between 12 and 15 weeks and there was an equal distribution of males and females. The animals arrived at least one week prior to initiation of the experiment.
All experiments were performed according to guidelines of The Danish Animal Experiments Council, the Danish Ministry of Justice.

*Treatments*

Haemophilia A mice receive either 75 or 300 mg/kg of recombinant human wild-type Factor VIIa or vehicle subcutaneous (5 ml/kg; for 300 mg/kg, 5 ml/kg injected at four sites), five minutes before amputation of the tail at a diameter of 3 mm. Mice treated with vehicle subcutaneous served as control. This vehicle contained (mg/ml): 15 NaCl, 5.15 $CaCl_2$, $2H_2O$, 1.55 Histidin, 0.5 Methionin, 0.07 Tween 80, 25 Mannitol and 10 Sucrose. The pH was 5.5.

*Tail bleeding assay*

Mice were anaesthetised with pentobarbital (100 mg/kg, Nomeco) and placed on a heating path to maintain body temperature. Bleeding was induced by amputating the tail at a diameter of 3 mm with a sharp scapular. Ten minutes prior to the amputation the tail was placed in 14 mL heated saline (37°C). The bleeding volume was then collected over a period of 20 minutes by intervals of 5 minutes. The blood loss was determined by quantifying the amount of haemoglobin. In brief, the erythrocytes were isolated by centrifugation at 4000 x *g* for five minutes. The supernatant was discarded and the cells were lysed with haemoglobin reagent (ABX Lysebio, HORIBA ABX). Cell debris was removed by centrifugation at 4000 x *g* for 30 sec. The samples were read at 405 nm and the total amount of haemoglobin were determined from calibrator rows of humane haemoglobin (Haemoglobin standarder (J:T Baker 3074)).

*Data analysis*

The data are presented as mean ± SEM and compared with either a one-way ANOVA (total blood loss) or two-way ANOVA (bleeding profiles).

Results

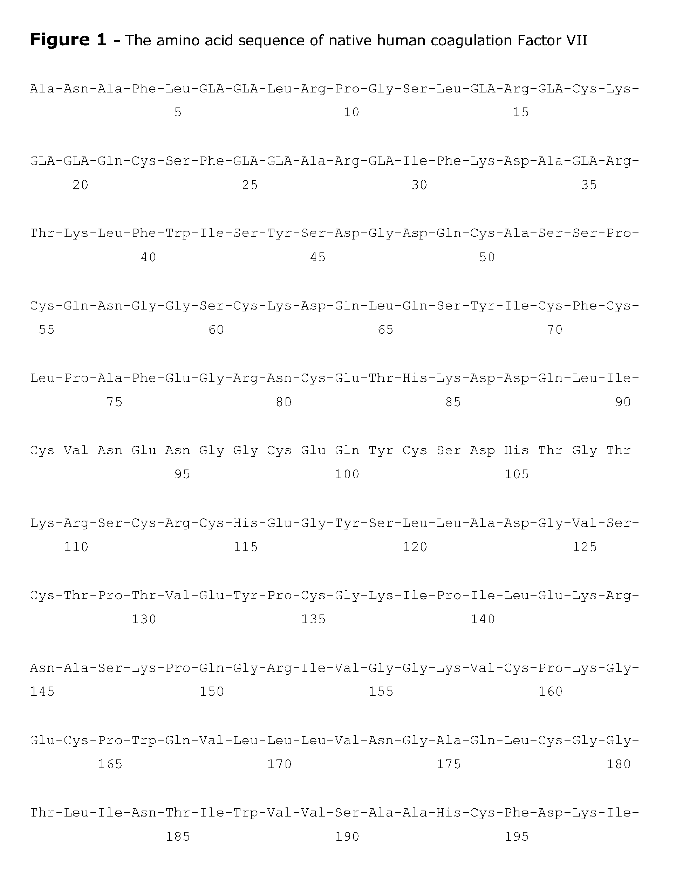

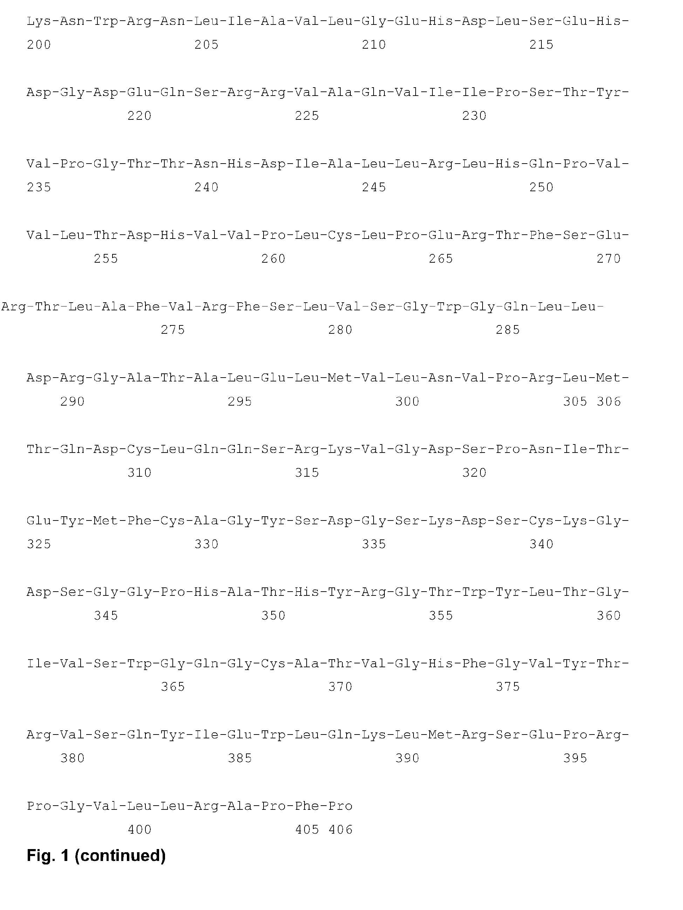

A significant effect of recombinant human wild-type Factor VIIa was observed on the bleeding profile 0-20 minutes after amputation of part of the tail. The bleeding profile is shown in figure 1. The total blood loss was reduced by 39 and 40% for 75 and 300 mg/kg of recombinant human wild-type Factor VIIa, respectively when compared to vehicle treatment administered subcutaneous. For comparison the maximal effect of intravenous FVIIa which is achieved with 15 mg/kg is shown in figure 2. This dose causes a 52% reduction in the total blood loss when compared to vehicle treatment administered intravenous.

All references, including publications, patent applications, and patents, cited herein are hereby incorporated by reference in their entirety and to the same extent as if each reference were individually and specifically indicated to be incorporated by reference and were set forth in its entirety herein (to the maximum extent permitted by law), regardless of any separately provided incorporation of particular documents made elsewhere herein.

Unless otherwise stated, all exact values provided herein are representative of corresponding approximate values (e.g., all exact exemplary values provided with respect to a particular factor or measurement can be considered to also provide a corresponding approximate measurement, modified by "about," where appropriate).

EMBODIMENTS OF THE INVENTION

1. Use of a FVIIa polypeptide for the preparation of a pharmaceutical formulation comprising said Factor VIIa polypeptide together with a pharmaceutically acceptable carrier for the on-demand treatment of a bleeding episode, wherein the pharmaceutical formulation is for subcutaneous or intramuscular administration in a single, high dose of said Factor VIIa polypeptide in a volume less than 1 ml.

2. Use according to embodiment 1, wherein the concentration of said FVIIa polypeptide is about 0.1 mg/ml to about 200 mg/ml, such as about 1 mg/ml to about 50 mg/ml.

3. Use according to any one of the embodiments 1-2, wherein the pH of the formulation is about 5.0 to about 7.0.

4. Use according to any one of the embodiments 1-3, wherein said FVIIa polypeptide is administered via a subcutaneous route.

5. Use according to any one of the embodiments 1-4, wherein said bleeding episode is associated with haemophilia.

6. Use according to any one of the embodiments 1-4, wherein said volume is less than 0.8 ml, such as less than 0.6 ml, such as less than 0.4 ml, such as less than 0.2 ml.

7. A method for treating an on-demand bleeding episode, the method comprising administering to a patient in need of such treatment an effective amount for such treatment of a Factor VIIa polypeptide, wherein the administering is via a subcutaneous or intramuscular route in a single, high dose of said Factor VIIa polypeptide in a volume less than 1 ml.

8. Method according to embodiment 7, wherein the concentration of said FVIIa polypeptide is about 0.1 mg/ml to about 200 mg/ml, such as about 1 mg/ml to about 50 mg/ml.

9. Method according to any one of the embodiments 7-8, wherein the pH of the formulation is about 5.0 to about 7.0.

10. Method according to any one of the embodiments 7-9, wherein said FVIIa polypeptide is administered via a subcutaneous route.

11. Method according to any one of the embodiments 7-10, wherein said bleeding episode is associated with haemophilia.

12. Method according to any one of the embodiments 7-11, wherein said volume is less than 0.8 ml, such as less than 0.6 ml, such as less than 0.4 ml, such as less than 0.2 ml.

**Claims**

1. Use of a FVIIa polypeptide for the preparation of a pharmaceutical formulation comprising said Factor VIIa polypeptide together with a pharmaceutically acceptable carrier for the on-demand treatment of a bleeding episode, wherein the pharmaceutical formulation is for subcutaneous or intramuscular administration in a single, high dose of said Factor VIIa polypeptide in a volume less than 1 ml.

2. Use according to claim 1, wherein the concentration of said FVIIa polypeptide is about 0.1 mg/ml to about 200 mg/ml, such as about 1 mg/ml to about 50 mg/ml.

3. Use according to any one of the claims 1-2, wherein the pH of the formulation is about 5.0 to about 7.0.

4. Use according to any one of the claims 1-3, wherein said FVIIa polypeptide is administered via a subcutaneous route.

5. Use according to any one of the claims 1-4, wherein said bleeding episode is associated with haemophilia.

6. Use according to any one of the claims 1-4, wherein said volume is less than 0.8 ml, such as less than 0.6 ml, such as less than 0.4 ml, such as less than 0.2 ml.

7. A method for treating an on-demand bleeding episode, the method comprising administering to a patient in need of such treatment an effective amount for such treatment of a Factor VIIa polypeptide, wherein the administering is via a subcutaneous or intramuscular route in a single, high dose of said Factor VIIa polypeptide in a volume less than 1 ml.

8. Method according to claim 7, wherein the concentration of said FVIIa polypeptide is about 0.1 mg/ml to about 200 mg/ml, such as about 1 mg/ml to about 50 mg/ml.

9. Method according to any one of the claims 7-8, wherein the pH of the formulation is about 5.0 to about 7.0.

10. Method according to any one of the claims 7-9, wherein said FVIIa polypeptide is administered via a subcutaneous route.

11. Method according to any one of the claims 7-10, wherein said bleeding episode is associated with haemophilia.

12. Method according to any one of the claims 7-11, wherein said volume is less than 0.8 ml, such as less than 0.6 ml, such as less than 0.4 ml, such as less than 0.2 ml.

**Figure 1** - The amino acid sequence of native human coagulation Factor VII

Ala-Asn-Ala-Phe-Leu-GLA-GLA-Leu-Arg-Pro-Gly-Ser-Leu-GLA-Arg-GLA-Cys-Lys-

GLA-GLA-Gln-Cys-Ser-Phe-GLA-GLA-Ala-Arg-GLA-Ile-Phe-Lys-Asp-Ala-GLA-Arg-

Thr-Lys-Leu-Phe-Trp-Ile-Ser-Tyr-Ser-Asp-Gly-Asp-Gln-Cys-Ala-Ser-Ser-Pro-

Cys-Gln-Asn-Gly-Gly-Ser-Cys-Lys-Asp-Gln-Leu-Gln-Ser-Tyr-Ile-Cys-Phe-Cys-

Leu-Pro-Ala-Phe-Glu-Gly-Arg-Asn-Cys-Glu-Thr-His-Lys-Asp-Asp-Gln-Leu-Ile-

Cys-Val-Asn-Glu-Asn-Gly-Gly-Cys-Glu-Gln-Tyr-Cys-Ser-Asp-His-Thr-Gly-Thr-

Lys-Arg-Ser-Cys-Arg-Cys-His-Glu-Gly-Tyr-Ser-Leu-Leu-Ala-Asp-Gly-Val-Ser-

Cys-Thr-Pro-Thr-Val-Glu-Tyr-Pro-Cys-Gly-Lys-Ile-Pro-Ile-Leu-Glu-Lys-Arg-

Asn-Ala-Ser-Lys-Pro-Gln-Gly-Arg-Ile-Val-Gly-Gly-Lys-Val-Cys-Pro-Lys-Gly-

Glu-Cys-Pro-Trp-Gln-Val-Leu-Leu-Leu-Val-Asn-Gly-Ala-Gln-Leu-Cys-Gly-Gly-

Thr-Leu-Ile-Asn-Thr-Ile-Trp-Val-Val-Ser-Ala-Ala-His-Cys-Phe-Asp-Lys-Ile-

```
Lys-Asn-Trp-Arg-Asn-Leu-Ile-Ala-Val-Leu-Gly-Glu-His-Asp-Leu-Ser-Glu-His-
200                 205                 210                 215


Asp-Gly-Asp-Glu-Gln-Ser-Arg-Arg-Val-Ala-Gln-Val-Ile-Ile-Pro-Ser-Thr-Tyr-
            220                 225                 230


Val-Pro-Gly-Thr-Thr-Asn-His-Asp-Ile-Ala-Leu-Leu-Arg-Leu-His-Gln-Pro-Val-
235                 240                 245                 250


Val-Leu-Thr-Asp-His-Val-Val-Pro-Leu-Cys-Leu-Pro-Glu-Arg-Thr-Phe-Ser-Glu-
            255                 260                 265                 270


Arg-Thr-Leu-Ala-Phe-Val-Arg-Phe-Ser-Leu-Val-Ser-Gly-Trp-Gly-Gln-Leu-Leu-
                275                 280                 285


Asp-Arg-Gly-Ala-Thr-Ala-Leu-Glu-Leu-Met-Val-Leu-Asn-Val-Pro-Arg-Leu-Met-
    290                 295                 300                 305 306


Thr-Gln-Asp-Cys-Leu-Gln-Gln-Ser-Arg-Lys-Val-Gly-Asp-Ser-Pro-Asn-Ile-Thr-
            310                 315                 320


Glu-Tyr-Met-Phe-Cys-Ala-Gly-Tyr-Ser-Asp-Gly-Ser-Lys-Asp-Ser-Cys-Lys-Gly-
325                 330                 335                 340


Asp-Ser-Gly-Gly-Pro-His-Ala-Thr-His-Tyr-Arg-Gly-Thr-Trp-Tyr-Leu-Thr-Gly-
        345                 350                 355                 360


Ile-Val-Ser-Trp-Gly-Gln-Gly-Cys-Ala-Thr-Val-Gly-His-Phe-Gly-Val-Tyr-Thr-
            365                 370                 375


Arg-Val-Ser-Gln-Tyr-Ile-Glu-Trp-Leu-Gln-Lys-Leu-Met-Arg-Ser-Glu-Pro-Arg-
    380                 385                 390                 395


Pro-Gly-Val-Leu-Leu-Arg-Ala-Pro-Phe-Pro
        400                 405 406
```

**Fig. 1 (continued)**

**Figure 2**

**Figure 3**

**EUROPEAN SEARCH REPORT**

Application Number

EP 07 11 2277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/82943 A (NOVO NORDISK AS [DK]; JOHANNESSEN MARIE [DK]; NORDFANG OLE JUUL [DK];) 8 November 2001 (2001-11-08) <br> * claims 1,3,7-9,13-15 * <br> * page 6, lines 2-24 * <br> * page 8, lines 22-32 * <br> * page 9, lines 17-21 * <br> * page 10, lines 11-25 * <br> ----- | 1-12 | INV. <br> A61K38/36 <br> A61P7/04 |
| Y | US 2005/032690 A1 (ROJKJAER LISA PAYNE [DK] ET AL) 10 February 2005 (2005-02-10) <br> * claims 1-9 * <br> * paragraphs [0088], [0089] * <br> ----- | 1-12 | |
| Y | WO 2004/000366 A (NOVO NORDISK AS [DK]; KLAUSEN NIELS KRISTIAN [DK]; BJOERN SOEREN [DK];) 31 December 2003 (2003-12-31) <br> * claims 1,9,11-13,16-22,24-28 * <br> * page 36, line 21 * <br> ----- | 1-12 | |
| Y | US 2002/137673 A1 (PINGEL HANS KURT [DK] ET AL) 26 September 2002 (2002-09-26) <br> * claims 1,8,25-28 * <br> * paragraph [0089] * <br> ----- | 1-12 | TECHNICAL FIELDS SEARCHED (IPC) <br><br> A61K |
| Y | US 2003/104978 A1 (PERSSON EGON [SE] ET AL) 5 June 2003 (2003-06-05) <br> * claims 1,36-38 * <br> * paragraphs [0200], [0201] * <br> ----- | 1-12 | |

The present search report has been drawn up for all claims

| Place of search | Date of completion of the search | Examiner |
|---|---|---|
| Munich | 3 December 2007 | Peris Antoli, Berta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 07 11 2277

This annex lists the patent family members relating to the patent documents cited in the above-mentioned European search report.
The members are as contained in the European Patent Office EDP file on
The European Patent Office is in no way liable for these particulars which are merely given for the purpose of information.

03-12-2007

| Patent document cited in search report | | Publication date | Patent family member(s) | | Publication date |
|---|---|---|---|---|---|
| WO 0182943 | A | 08-11-2001 | AU | 5614801 A | 12-11-2001 |
| | | | EP | 1280548 A2 | 05-02-2003 |
| | | | JP | 2003531862 T | 28-10-2003 |
| US 2005032690 | A1 | 10-02-2005 | NONE | | |
| WO 2004000366 | A | 31-12-2003 | AU | 2003240439 A1 | 06-01-2004 |
| | | | BR | 0311978 A | 22-03-2005 |
| | | | EP | 1517710 A1 | 30-03-2005 |
| | | | JP | 2006513142 T | 20-04-2006 |
| | | | MX | PA04012496 A | 12-09-2005 |
| | | | US | 2005113565 A1 | 26-05-2005 |
| US 2002137673 | A1 | 26-09-2002 | US | 2005075289 A1 | 07-04-2005 |
| US 2003104978 | A1 | 05-06-2003 | US | 2003100740 A1 | 29-05-2003 |
| | | | US | 2006252129 A1 | 09-11-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6310183 B
- US 6833352 B
- WO 9307890 A
- WO 9526750 A
- WO 9528954 A
- US 4784950 A
- WO 0331464 A
- US 20040043446 A
- US 20040063911 A
- US 20040142856 A
- US 20040137557 A
- US 20040132640 A
- WO 0104287 A
- US 20030165996 A
- WO 0158935 A
- WO 0393465 A
- WO 0202764 A
- US 20030211094 A
- US 5580560 A
- DK 0200189 W
- WO 02077218 A
- WO 0238162 A
- WO 9920767 A
- US 6017882 A
- US 6747003 B
- US 20030100506 A
- WO 0066753 A
- US 20010018414 A
- US 2004220106 A

- US 200131005 B
- US 6762286 B
- US 6693075 B
- US 6806063 B
- US 20030096338 A
- WO 04029091 A
- WO 0183725 A
- WO 0222776 A
- DK 0200635 W
- WO 03027147 A
- DK PA200201423
- WO 04029090 A
- DK PA200101627
- JP 2001061479 B
- EP 200421 A
- EP 06120000 A
- WO 2006013202 A
- WO 2006035057 A
- WO 2005014049 A
- WO 2005035553 A2
- WO 9520039 A
- WO 9838285 A
- WO 2005070468 A
- WO 2006013202 A2
- WO 2002077218 A
- EP 2006063310 W
- US 4456591 A
- WO 2005023308 A

**Non-patent literature cited in the description**

- **LINO et al.** *Arch. Biochem. Biophys.,* 1998, vol. 352, 182-192
- **MOLLERUP et al.** *Biotechnol. Bioeng.,* 1995, vol. 48, 501-505
- **KORNFELT et al.** *Arch. Biochem. Biophys.,* 1999, vol. 363, 43-54
- **HAGEN et al.** *Proc.Natl.Acad.Sci. USA,* 1986, vol. 83, 2412-2416
- **TAKEYA et al.** *J. Biol. Chem.,* 1988, vol. 263, 14868-14872
- **BROZE ; MAJERUS.** *J. Biol.Chem.,* 1980, vol. 255 (4), 1242-1247

- **HEDNER ; KISIEL.** *J. Clin.Invest.,* 1983, vol. 71, 1836-1841
- **OSTERUD et al.** *Biochem.,* 1972, vol. 11, 2853
- **HEDNER et al.** *J. Clin. Invest.,* 1983, vol. 71, 1836
- **WAN ; LEE.** *Journal of Pharm Sci,* 1974, vol. 63, 136
- Remington: The Science and Practice of Pharmacy. 1995